# EUROPEAN PATENT APPLICATION

(11) **EP 1 070 450 A1**
(43) Date of publication of application: **24.01.2001**
(21) Application number: 00305872.4
(22) Date of filing: 12.07.2000
(51) Int. Cl.: A01H 4/00

(54) **Method for plant tissue culture**

(30) Priority: 21.07.1999 JP 20655399
(71) Applicant: NISSHINBO INDUSTRIES, INC., Chuo-ku, Tokyo (JP)
(72) Inventor: Hasegawa, Osamu c/o Nisshinbo Industries, Inc., Chiba-shi, Chiba 267-0056 (JP); Ohno, Yojiro c/o Nisshinbo Industries, Inc., Chiba-shi, Chiba 267-0056 (JP)
(74) Representative: Bannerman, David Gardner

(57) **Abstract**

Plant tissue is cultured by using a plant tissue culture support material with density of 0.01 to 2 g/cm³ which is comprised of vermiculite of average particle diameter of 0.1 to 10 mm and cellulose fiber of average fiber length of 0.01 to 5 mm at a mixing ratio in weight of the vermiculite and the cellulose fiber being 5 : 95 to 95 : 5, and a culture broth with addition of a germicide at such an amount that the germicide itself can singly prevent contamination. Thus, there is provided a method for plant tissue culture with no laborious sterilization process or with no need of sterile transplantation of the plant tissue.

## Description

### BACKGROUND OF THE INVENTION

### Field of the Invention

The present invention relates to a method for plant tissue culture; more specifically, the invention relates to a method for plant tissue culture with no laborious sterilization process, the method comprising the step of propagating, acclimatizing and growing plant tissue into mature seedling in a simple and efficient manner.

### Description of the Related Art

Plant tissue culture technology is an essential technology from the respect of fundamental research works about gene expression and metabolism and from the practical and industrial respect to obtain a plant with homogenous and excellent characters by growing the tissue or cell of the plant at a mass scale. This plant regeneration process by plant tissue culture generally comprises the following scheme. That is, for higher plants, a plant cell or plant tissue as a genetic source is maintained by primary culture or subculture; the plant cell or plant tissue is then induced into plantlets or multiple axillary shoots by early-stage branching method, protocorm-like body method and shoot primordium method, which serves as a step for mass-scale production. The resulting plantlets and multiple axillary shoots are grown into plantlets with stems and leaves by culturing for seedling preparation; and subsequently, adventitious roots are differentiated.

For such plant tissue culture, conventionally, photomixotrophic method comprising the use of gelling agents such as agar and Gelrite as culture medium materials and the addition of saccharides as a carbon source to the culture medium has been used routinely.

However, such method has the following problems.
1. Sterilization procedure is required because contamination occurs due to the saccharides in the culture medium.
2. Root growth is retarded and the resulting root is aquatic root, because the culture medium (gel) contains no gas phase.
3. Leave growth is poor because most of the growth depends on the saccharides in the culture medium while the dependency thereof on photosynthesis is at a low ratio.

Regarding the problem 1, in particular, the culture medium, the culture vessels and the inner culturing environment are required to be sufficiently sterilized by autoclave and the like; additionally, the transplantation or subculturing procedure of the plant tissue should be carried out aseptically in clean bench and the like with sufficient care; even thereafter, contamination should be avoided as much as possible. Such procedures are laborious and take a long period of time. Therefore the method has a number of problems so that it makes the mass-scale plant production by such plant tissue culture inefficient.

Concerning the problem 2, additionally, root induction is difficult in gel-like culture media in most cases; even when root growth is observed, the resulting root never functions in the nursery soil during acclimatization, so the growth of a fresh root suitable for the nursery soil is required for the growth. Therefore, the production yield of the plant is then poor, so disadvantageously.

Concerning the problem 3, furthermore, the laborious acclimatization step requiring a long period of time is indispensable until the plant can grow photoautotrophically; additionally, plants disable to adapt to such acclimatization are dead. Hence, the production yield decreases, disadvantageously.

So as to improve these problems, several methods have been proposed. For example, methods using germicides as the methods for overcoming the problem 1 have been reported (Japanese Patent Laid-open (Kokai) 4-311326, Japanese Patent Laid-open (Kokai) 9-235208, Japanese Patent Laid-open (Kokai) 7-255305, Japanese Patent Laid-open (Kokai) 7-255306, Japanese Patent Laid-open (Kokai) 3-297331 and Japanese Patent Laid-open (Kokai) 10-313717).

The Japanese Patent Laid-open (Kokai) 4-311326 discloses a method for culturing Cyclamen in a culture medium containing imidazole-series or triazole-series germicides. However, the use of such germicides therein is just for the purpose of eliminating Cyclamen-specific symbiosis bacteria in the tissue. Additionally, the method is based on the premise of the use of saccharide in the culture medium and lists only the use of agar as a gelling agent.

The Japanese Patent Laid-open (Kokai) 9-235208 discloses a system prepared by adding germicides such as isocyanur chloride and hypochlorite salt to an agar medium. Nevertheless, the purpose of the invention resides in "providing a culture medium with no need of costly high-pressure steam sterilizer" not in the simplification or efficiency of the culture procedures. Thus, the system requires conventional aseptic methods afterwards or laborious procedures such as spraying of aqueous hypochlorite solution or aqueous isocyanur chloride solution, although the sterilization procedure of the culture medium can be cutted out by the system. The application suggests a possibility of the use of gelling agents other than agar used as a support material in only one example thereof, but never includes any description of other support materials. Furthermore, the culture medium is based on the premise of the use of saccharide, with no idea of non-saccharide culturing.

The Japanese Patent Laid-open (Kokai) 7-255305 discloses a culture method comprising adding an antibacterial agent to a non-saccharide culture medium adjusted to pH 3.0 to 4.5 under controls of optical environment and gaseous environment. By the method, however, the pH 3.0 to 4.5 is the essential condition. No effect is observed around pH 5.8 for use in general plant tissue culture. Additionally, the method may have a problem that the plant tissue therein poorly grows and is frequently dead because the pH of the culture medium is adjusted to 3.0 to 4.5.

The Japanese Patent Laid-open (Kokai) 7-255306 discloses a culture method in a non-saccharide culture medium with addition of a given concentration of hypochlorite salt under controls of optical environment and gaseous environment. However, the application never encompasses the idea of no autoclave sterilization of the culture vessel or culture medium to be used for culture, so the application includes no description of any procedure therefor. Additionally, the examples include only the description of a method comprising sterilizing the culture vessel and culture medium in autoclave and thereafter effecting culturing. In other words, the invention is for the purpose of preventing contamination and never makes any contribution to the purpose of cutting out laborious sterilization procedure for efficient culturing. Thus, the invention does not serve as means for solving the problems.

The Japanese Patent Laid-open (Kokai) 3-297331 discloses a culture medium prepared by adding highly adsorbent materials such as active charcoal to a porous or fibrous culture medium material and adding then a germicide hardly soluble in cold water and highly soluble in hot water to the resulting culture medium material. The invention is based on an improvement of the technique based on culturing with saccharide as a conventional method, with not any suggestion of the application of non-saccharide culture method. In the invention, furthermore, the addition of absorbent substances such as active charcoal and zeolite is essential. Hence, the preparation of the culture medium is laborious. Additionally, a complicated procedure of depositing germicides by cooling the culture medium after dissolving the germicides in it under heating is required, disadvantageously.

The Japanese Patent Laid-open (Kokai) 10-313717 discloses a culture method of tomato seedling with addition of silver compounds as germicides. However, silver compounds are not only costly but also burdensome for humans and environment. Therefore, the method is not necessarily advantageous.

An additional report has been issued as a method for overcoming the problem 2, telling that the usage of a plant support material containing vermiculite and cellulose fiber results in very good rooting, thus it enables to obtain healthy seedlings that do not require acclimatization only after the first culture and such seedlings can then be transplanted in open field nursery soil as they are (WO 97/48271). The publication describes examples with addition of germicides or antibacterial agents so as to reduce contamination, but includes not any description about cutting out laborious sterilization procedure, or not any description about no need of sterile transplantation of plants and not any description about an ability to propagate, to acclimatize and to grow plant tissue into mature seedling in a simple and an efficient manner.

### SUMMARY OF THE INVENTION

From such respects, the present invention has been accomplished. More specifically, it is an object of the present invention to provide a method for plant tissue culture with no need of sterilization of culture medium, culture vessel and the like or with no need of sterile transplantation of the plant tissue.

The inventors of the present invention assiduously studied in order to achieve the aforementioned object. As a result, they found that plant culture on a mixture of vermiculite and cellulose fiber used as a support material filled, with a culture broth including a germicide does not require the sterilization of the culture medium and culture vessel or the sterile transplantation of the plant tissue. Because the seedling obtained in accordance with the present invention is excellent in terms of root growth, plant growth and plant yield and can be transplanted as it is for acclimatization with no removal of the support material, the acclimatization process can be simplified or cutted out, involving the shortening of the period required for growing into mature seedling. Thus, the present invention has been achieved.

More specifically, the present invention relates to a method for plant tissue culture, comprising the step of culturing plant tissue by using a plant tissue culture support material at a density of 0.01 to 2 g/cm³, which is comprised of vermiculite of average particle diameter of 0.1 to 10 mm and cellulose fiber of average fiber length of 0.01 to 5 mm at a mixing ratio in weight of the vermiculite and the cellulose fiber being 5 : 95 to 95 : 5, and a culture broth with addition of a germicide at such an amount that the germicide itself can singly prevent contamination.

Additionally, the present invention provides a method for plant tissue culture, wherein the culture broth does not contain carbon source.

Still additionally, the present invention provides a method for plant tissue culture, wherein the germicide is a mixture of one or two or more selected from the group consisting of chlorine germicides and azotic germicides.

Still more additionally, the present invention provides a method for plant tissue culture, wherein the germicide is a mixture of one or two or more selected from the group consisting of hypochlorite salts, iprodion and benomyl.

And yet additionally, the present invention provides a method for plant tissue culture, wherein the effective concentration of the hypochloric acid is 0.001 to 0.05 %, the concentration of the iprodion is 0.001 to 0.1 % and that of the benomyl is 0.001 to 0.1 %.

The present invention furthermore provides a method for plant tissue culture, wherein the step of culturing plant tissue is performed under light at a photosynthetic photon flux of 50 to 500 µmol/m²/sec while maintaining carbon dioxide gas concentration in culture environment to be at 300 to 2000 µmol/mol.

### DETAILED DESCRIPTION OF THE PREFERRED EMBODIMENTS

The present invention will be described in detail below.

The present invention relates to a method for plant tissue culture, comprising the step of culturing plant tissue by using a plant tissue culture support material at a density of 0.01 to 2 g/cm³, which is comprised of vermiculite of average particle diameter of 0.1 to 10 mm and cellulose fiber of average fiber length of 0.01 to 5 mm at a mixing ratio in weight of the vermiculite and the cellulose fiber being 5 : 95 to 95 : 5, and a culture broth with addition of a germicide at such an amount that the germicide itself can singly prevent contamination.

In the present invention, the vermiculite is satisfactorily of any type, with no specific limitation, but is preferably of average particle diameter of 0.1 to 10 mm.

When the particle diameter of the vermiculite is too small, the effect thereof to retain the culture broth is reduced, involving the hardening of the support material, which sometimes causes difficulty in the insertion of plant tissue. When the particle diameter of the vermiculite is too large, it is difficult to insert plant tissue at an arbitrary position in the support material, with the resultant poor processability.

Furthermore, vermiculite preliminarily subjected to various treatments may be used. Examples of such treatments include chemical and physical treatments such as heating treatment, cooling treatment, purification treatment, swelling treatment, pulverization treatment, granulating treatment, impregnation treatment, coating treatment and so forth.

The cellulose fiber is preferably of average fiber length of 0.01 to 5 mm.

When the fiber length of the cellulose fiber is too short, the support material is hardened, which sometimes causes difficulty in the insertion of plant tissue. When the fiber length of the cellulose fiber is too long, it is likely to be difficult of the uniform mixing of the vermiculite and the cellulose fiber, because of few tangling to each other.

The cellulose fiber is of any type, with no specific limitation. Cotton lint, cotton linter, softwood cellulose, hardwood cellulose, bast cellulose, hemp cellulose, regenerated cellulose and bacteria cellulose, and mixtures thereof, may be used.

Furthermore, these cellulose fibers preliminarily subjected to various treatments may be used. The various treatments herein referred to include chemical and physical treatments, such as heating treatment, cooling treatment, purification treatment, decrystallization treatment, swelling treatment, treatment for reducing the degree of polymerization, treatment for derivatization, cross-linking treatment, treatment for converting crystal form, regeneration treatment by dissolution, pulverization treatment, granulating treatment, impregnation treatment, coating treatment and so forth.

The support material may optionally be added with activated carbon, coconut shell fiber, perlite, smoked charcoal, peat moss and so forth.

The mixing ratio of the vermiculite and the cellulose fiber is preferably at a weight ratio of 5 : 95 to 95 : 5. When the cellulose fiber is below 5 % by weight, plant tissue is poorly fixed therein because the vermiculite is in particle. Above 95 % by weight, the resulting culture medium is hardened, with the resultant difficulty in the insertion of plant tissue.

The density of the support material is preferably 0.1 to 2 g/cm³. When the density is above the range, the culture medium gets hard even after a culture broth and the like are charged, sometimes involving difficulty in the insertion of plant tissue and also causing poor root growth. When the density is below the range, plant tissue is poorly fixed.

In the present invention, the term "plant tissue" refers to plant tissue per se for use in the culture of the cells or the culture of meristem of higher plants. Plantlets and multiple axillary shoots are included in the resulting products generated by primary culture and subculture and formed by early-stage branching method, protocorm-like body method and shoot primordium method.

The plant type to be cultured is with no limitation and includes for example all herbs, flowers, trees, such as Cattleya, Phalaenopsis, Dendrobium, Cymbidium, Paphiopedilum, Vanda, Ascoscenda, Epidendrum, Miltonia, Oncidium, Odontglossum, Epiphlonitis, Calanthe, Nephrolepis, Dieffenbachia, Pecteilis, Cymbidium, Burceraceae, Syngonium, Streptocarpus, clematis, geranium, poinsettia, rhododendron, gloxinia, Alstroemeria, Hemerocallis, freesia, iris, carnation, Gypsophila elegans, statice, chrysanthemum, gerbera, primula, Saintpaulia, Cyclamen, lily, gladiolus, dahlia, rose, Bouvardia, azalea, gentian, narcissus, amaryllis, hyacinth, Begonia, aster savatieri, Miltonia, Asplenium, Benjamin, Spathiphyllum, Epipremnum aureum, Alocasia, Monstera, Philodendron, Syndabsis, Caladium, Ananas, Neoregelia, Dracaena, Cyathea, Adiantum, Asplenium nidus, Pteridophyte, Anthurium, Zoysia, strawberry, garlic, Japanese horseradish, cucumber, tomato, egg plant, potato, sweet potato, aroid, yam, Chinese yam, carrot, melon, konjak, bog rhubarb, asparagus, Brassica, Oryzae, barley, cotton plant, kenaf, banana, pineapple, oil palm, coffee, cocoa, apple, pear, Japanese persimmon, grape, peach, Japanese apricot, citrus, Japanese tea, raspberry, blue berry, almond, cherry, Litchi, mangostin, senkyu, Pinellia ternata, Jiou, Atractylodes japonica, Belladonna, aconite, Scopolia, ipecac, sialid, rhubarb, cherry blossom, kozo, white birch, Abelia, Eucalyptus, acacia, rubberwood, Paulonia, Populus, aspen, Sandalwood, Tectona, Latania, elm, birch, mulberry, oak species, hiba, cedar, cypress, Picea, fir, pine, yew, sequoia, lauan, Dipterocarpaceae, gmelina, and mahogany.

General culture broths for plant tissue culture are used as the culture broth in the present invention. More specifically, all culture broths such as MS medium, White's medium, Vicin & Went medium, Hyponex medium, and Woody Plant medium are applicable. Furthermore, these culture broths may be modified corresponding with the used plant type, as required, in such manners that the concentrations of the culture broths may be reduced or that certain types of ingredients such as phosphoric acid and iron or various plant hormones may be added to the culture broths.

In the method for plant tissue culture of the present invention, culturing is preferably performed in a culture broth with no content of carbon source. Therefore contamination due to the presence of carbon source can be prevented, and additionally, photoautotrophic growth is permitted because the carbon source for plant growth is obtained from carbon dioxide in the culture environment. In such manner, a healthy plant with good root growth and large leaf area can be yielded, so acclimatization process can be simplified or cutted out. Herein, the term "carbon source" refers to a carbon source possibly assimilated by plants and includes saccharides such as sucrose, glucose, fructose and so forth.

In the present invention, any germicide capable of efficiently preventing contamination without any adverse influence on plant, growth is preferably used. In such sense, a mixture of one or two or more selected from the group consisting of chlorine germicides and azotic germicides are preferably used. Hypochlorite salts are preferable as the chlorine germicide, benomyl and KK-681 (produced by K·I Chemical Industry Co.Ltd.) are preferable as the azotic germicide. Iprodion belonging to the chlorine germicide and the azotic germicide may be also preferably used. As to the concentrations of these germicides to be added to the culture medium, the minimum concentration capable of efficiently preventing the appearance of the contamination is satisfactory. The effective concentration of the hypochloric acid is 0.001 to 0.05 %, concentration of the iprodion is 0.001 to 0.1 %, that of the benomyl is 0.001 to 0.1 % and that of the KK-681 is 0.01 to 1.0 %, preferably. At concentrations below the ranges, no sterilization effects can be observed, while above the ranges, the germicides adversely affect plants, unpreferably.

The addition of germicides to the culture medium enables to cut out the conventional sterilization procedure of autoclave; and no deterioration of the culture medium by sterilization at high temperature and high pressure is induced, so the plant grows extremely well. For acclimatization, the plant can be transplanted as it is with no removal of the support material. The acclimatization process can be simplified or cutted out to shorten the period for growing into mature seedling; additionally, at an early acclimatization stage or an early stage of the plant directly placed in a farm, the presence of germicides around the plant can suppress the proliferation of contaminating germs for a while, so the efficiency of the plant acclimatizing to the environment is so enhanced that the resulting plant grows extremely well.

The light conditions and gas conditions for growing plant tissue are satisfactorily the conventional conditions for use in the photomixotrophic growth. Conditions for promoting the photoautotrophic growth are preferable.

When the conditions that the carbon dioxide concentration is 300 to 2000 µmol/mol and the photosynthetic photon flux is 50 to 500 µmol/m²/sec are satisfied, the growth of plant tissue is more enhanced, involving prominent growth of the roots and leaves. When the carbon dioxide concentration and the photosynthetic photon flux are below the ranges, the effects on the growth are reduced, which is unpreferably. When the carbon dioxide concentration and the photosynthetic photon flux are above the ranges, it sometimes affects the growth of plants in an inhibitory fashion, and which, is unpreferably.

According to the culture method of the present invention, the culture medium, the culture vessel and the culture environment are not required to be sterilized in autoclave, so laborious processes can be cutted out. Furthermore, plant tissue can be placed in the culture medium with no need of the procedure to be operated in sterile environment, so any specific equipment such as clean bench is not needed.

It is not necessary to aseptically prepare the culture medium. The preparation may be carried out as follows.

The culture medium (support material) described in the present invention is set in a culture vessel in non-sterile environment. It is not required that these are sterilized. There are no restrictions on culture vessels, any culture vessel may be used. Thus, culture vessels for general use, including those made of glass, plastic, vinyl and the like can be used. These culture vessels may be of any size. Preferably, the culture vessels are transparent enough to readily permeate light. So as to control the carbon dioxide gas concentration and humidity, furthermore, containers attached with gas permeable membranes, for example Milliseal (manufactured by Millipore, Co.), may sometimes be used.

Additionally, a method comprising directly placing the support material in a culture chamber to grow plant tissue with no use of such culture vessel is also applicable. In this case, the support material is preferably placed in receptacle, tray, corrugated board, multi-celled tray and the like for good processability, from the respect of plant transplantation. A separately prepared culture broth is added to the support material. The culture broth is as described above, to which the germicide may be added to a predetermined concentration. Otherwise, the germicide may be added to the support material after the culture broth is added to the support material. In any case, no sterilization is required.

It is not required to be in sterile environment for placing plant tissue in the culture medium thus prepared. The procedure can satisfactorily be practiced in non-sterile environment. Accordingly, the procedure can be carried out in general laboratories and rooms such as green house, or outside. No specific equipment such as clean bench is needed.

The culture temperature depends on the plant type, but generally, the culture temperature is 20 °C to 35 °C. Additionally, the humidity condition is generally at 50 % to 100 %. The prevention of extra-moisture and extreme dryness is significant for plant growth.

Other culture conditions, for example type of light, direction of light irradiation, light and dark cycle, amount of culture broth, concentration of culture broth, can be set to the suitable culture conditions, by modifying the individual conditions.

The plant grown in accordance with the present invention is taken out of the culture vessel per support material, which is then placed in an acclimatization bed or directly placed in a farm. Then, the support material may be flushed with running water, but it is advantageous if the plant in the support material is transplanted as it is, considering any potential occurrence of damages on the root and laborious works.

Because the support material mainly comprises the vermiculite and cellulose components, no burden is loaded on the environment in which the plant is transplanted.

According to the present invention, good plant growth, good rooting and significant leaf growth can be realized, so the acclimatization process can be shortened or can sometimes be cutted out, compared with conventional methods. Due to cutting out the acclimatization process, the plant can directly be placed in a farm after the culturing and growing stages.

As described above, using the method of the present invention enables to cut out laborious sterilization procedures of culture medium, culture vessel and the like. Additionally, it is not required to transplant plant tissue aseptically. Furthermore, the deterioration of the culture medium because of sterilization under high-temperature and high-pressure conditions never occurs. Thus, the plant growth is extremely well. Additionally, the support material can be transplanted as it is with no need of removal for acclimatization, so the acclimatization process can be simplified or can be cutted out, involving higher yield. Hence, not only the period for growing into mature seedling can be shortened but also the presence of any germicide around the plant can suppress germ proliferation for a while, even at the primary stage of acclimatization or an early stage of the plant placed directly in a farm. Thus, the acclimatization efficiency of the plant to the environment can be enhanced, with the resultant plant of extremely good growth.

### Examples

The present invention will now be described more specifically in the following examples. However, the present invention is not limited to the following examples, unless departed from the spirit of the present invention.

### Preparation of culture media

### Example 1

A molded product (mixing ratio in weight = 5 : 1; density of 0.13 g/cm³ ; 5 × 5 × 2 cm; Florialite manufactured by Nisshinbo) of vermiculite (average particle diameter of 2 mm) and pulp (average fiber length of 1.2 mm) was placed in a Magenta Box (culture vessel). By adding an MS culture broth (without saccharide; 100 mg/l iprodion) of 40 ml to the molded product, a culture medium was completely prepared and was then placed on a laboratory bench. The time required for the procedure was 5 minutes.

### Comparative Example 1

A molded product (mixing ratio in weight = 5 : 1; density of 0.13 g/cm³ 5 × 5 × 2 cm; Florialite manufactured by Nisshinbo) of vermiculite (average particle diameter of 2 mm) and pulp (average fiber length of 1.2 mm) was placed in a Magenta Box (culture vessel). By adding 40 ml of an MS culture broth (without saccharide) to the molded product, the resulting culture medium was sterilized in an autoclave (121 °C for 20 minutes). The culture medium was taken out and placed in a clean bench. Because the elevation of the temperature in the autoclave and the cooling of the autoclave required a long time, the time required for the procedure was 2 hours.

### Plant tissue transplantation

### Example 2

One hundred samples of the culture medium of Example 1 were used. The top node (about 3 cm) of each aseptically cultured carnation seedling was inserted in each culture medium on a laboratory bench (non-sterile environment) and cultured at 25 °C for one month. Forceps or a surgery knife was used without sterilization. The time required for the procedure was 2 hours.

### Comparative Example 2

One hundred samples of the culture medium of Comparative Example 1 were used. The top node (about 3 cm) of each aseptically cultured carnation seedling was inserted in each culture medium in a clean bench and cultured at 25 °C for one month. The workers sufficiently disinfected their hands and arms with 70 % ethanol. Using forceps and a surgery knife preliminarily sterilized in an autoclave, the workers carried out the procedure. The workers took a break during the procedure; and prior to the re-start of the procedure, the workers again disinfected their hands and arms. The time required for the procedure was 4 hours.

### Plant tissue culture of Japanese horseradish

### Example 3 and Comparative Example 3

Japanese horseradish was grown and cultured by lateral shoot induction. The plant was divided into each shoot, which was then subjected to the test. Five shoots were placed in each plant box. For each test lot, 20 plant boxes (100 shoots in total) were used.

Florialite of Example 1 was used as the support material in the present invention. As a control, Gelrite was used.

The plant tissues in the autoclave lots were placed aseptically in a clean bench. The plant tissues in the non-autoclave lots were placed in non-sterile environment (on a laboratory bench).

Culture was continued for 6 weeks; thereafter, the plant tissues were placed on acclimatization beds. In case of Gelrite, the plants were placed in the nursery soil after the Gelrite was sufficiently flushed with running water; in case of Florialite, the support material was not rinsed and placed as it was on the acclimatization beds.

Conditions of the plant tissue culture of Japanese horseradish are shown below.
Plant: Japanese horseradish
Support material: Gelrite (2.5 g/l), Florialite (molded product of Example 1; 5 × 5 × 2 cm)
Culture vessel; Magenta Box (one Milliseal membrane attached on the lid part)
Germicide: sodium hypochlorite, iprodion, benomyl
Culture broth: MS culture broth (pH 5.8); 3 % sucrose added to MS culture broth when saccharide is contained
Culturing period: 6 weeks
Exposure to light: 16 hours in light and 8 hours in dark; 50 µmol/m²/sec
CO₂ concentration: 300 µmol/mol
Temperature: 20 °C

Experimental lots were defined as follows in Table 1.

**Table 1**

| Culture conditions of individual experimental lots Autoclave Gelrite lots |
|---|
| 1. with saccharide |
| 2. non-saccharide |

| Autoclave Florialite lots |
|---|
| 3. with saccharide |
| 4. non-saccharide |

| Non-autoclave Florialite lots |
|---|
| 5. with saccharide |
| 6. non-saccharide |
| 7. non-saccharide/ sodium hypochlorite : 0.005 % (effective chloride concentration) |
| 8. non-saccharide/ sodium hypochlorite : 0.01 % (effective chloride concentration) |
| 9. non-saccharide/ sodium hypochlorite : 0.1 % (effective chloride concentration) |
| 10. non-saccharide/ iprodion : 0.005 % |
| 11. non-saccharide/ iprodion : 0.01 % |
| 12. non-saccharide/ iprodion : 0.2 % |
| 13. non-saccharide/ benomyl : 0.005 % |
| 14. non-saccharide/ benomyl : 0.01 % |
| 15. non-saccharide/ benomyl : 0.2 % |
| 16. non-saccharide/ iprodion : 0.005 %, benomyl :0.005 sodium hypochlorite : 0.005 % (effective chloride concentration) |

The growth of the plant was assayed on the basis of the following items.

### 〈From the start of culture to 6-week later〉

The number of vessels with healthy plant growth ; the number of vessels contaminated with germs ; and the number of vessels with adverse drug influence.

### 〈After 6-week culture〉

Fresh weight of plant ; maximum petiole length ; root growth.

### 〈After transplantation on acclimatization bed〉

Survival ratio and growth.

The measurement was carried out as follows. Fresh weight of plant: The weights of all the plants with removal of the support material were measured and averaged per each test lot.
Maximum petiole length : The maximum length of stem branching point to leaf joint in each plant was measured; and the average thereof in the plants per each test lot was calculated.
Root growth : The degree of root growth was relatively assayed on the basis of the following 5 ranks;
   +++ : extremely great root growth
   ++ : good root growth
   + : normal root growth
   ± : poor root growth
   - : no root growth.
Survival ratio : The survival ratio of plant after acclimatization was expressed in percentage.
Growth : Growth was assayed on the basis of the following 4 ranks;, great, good, normal and delay.

Tables 2 and 3 show the results of growth after 6-week culture and acclimatization.

**Table 2**

| Growth state of Japanese horseradish after 6-week culture | | | |
|---|---|---|---|
| Test lots | Healthy vessel | Vessel contaminated with germs | Vessel with adverse drug influence |
| Autoclave Gelrite | | | |
| 1. with saccharide | 20 | 0 | 0 |
| 2. non-saccharide | 20 | 0 | 0 |

| Autoclave Florialite | | | |
|---|---|---|---|
| 3. with saccharide | 20 | 0 | 0 |
| 4. non-saccharide | 20 | 0 | 0 |

| Non-autoclave Florialite | | | |
|---|---|---|---|
| 5. with saccharide | 0 | 20 | 0 |
| 6. non-saccharide | 2 | 18 | 0 |
| 7. non-saccharide NaOCl: 0.005 % | 20 | 0 | 0 |
| 8. non-saccharide NaOCl: 0.01 % | 20 | 0 | 0 |
| 9. non-saccharide NaOCl: 0.1 % | 0 | 0 | 20 |
| 10. non-saccharide iprodion: 0.005 % | 20 | 0 | 0 |
| 11. non-saccharide iprodion: 0.01 % | 20 | 0 | 0 |
| 12. Non-saccharide iprodion: 0.2 % | 0 | 0 | 20 |
| 13. non-saccharide benomyl: 0.005 % | 20 | 0 | 0 |
| 14. non-saccharide benomyl: 0.01 % | 20 | 0 | 0 |
| 15. Non-saccharide benomyl: 0.2 % | 0 | 0 | 20 |
| 16. non-saccharide NaOCl: 0.005 %; iprodian: 0.005 %; benomyl: 0.005 % | 20 | 0 | 0 |

**Table 3**

| Growth results after culturing of Japanese horseradish for 6 weeks and subsequent acclimatization | | | | | |
|---|---|---|---|---|---|
| Test lots | Results of culture | | | Results of acclimati-zation | |
| | Fresh weight of plant in mg | Maximum petiole length in mm | Root growth | Survival ratio in % | Growth |
| Autoclave Gelrite | | | | | |
| 1. with saccharide | 340 | 45 | + | 50 | delay |
| 2. non-saccharide | 180 | 25 | ± | 10 | delay |

| Autoclave Florialite | | | | | |
|---|---|---|---|---|---|
| 3. with saccharide | 430 | 60 | ++ | 90 | good |
| 4. non-saccharide | 380 | 55 | ++ | 90 | good |

| Non-autoclave Florialite | | | | | |
|---|---|---|---|---|---|
| 5. with saccharide | - | - | - | - | - |
| 6. non-saccharide | - | - | - | - | - |
| 7. non-saccharide NaOCl: 0.005 % | 450 | 63 | +++ | 95 | great |
| 8. non-saccharide NaOCl: 0.01 % | 440 | 61 | +++ | 95 | great |
| 9. non-saccharide NaOCl: 0.1 % | - | - | - | - | - |
| 10. non-saccharide iprodion: 0.005 % | 460 | 65 | +++ | 95 | great |
| 11. non-saccharide iprodion: 0.01 % | 450 | 63 | +++ | 95 | great |
| 12. Non-saccharide iprodion: 0.2 % | - | - | - | - | - |
| 13. non-saccharide benomyl: 0.005 % | 450 | 63 | +++ | 95 | great |
| 14. non-saccharide benomyl: 0.01 % | 440 | 61 | +++ | 95 | great |
| 15. Non-saccharide benomyl: 0.2 % | - | - | - | - | - |
| 16. non-saccharide NaOCl: 0.005 % iprodion: 0.005 %, benomyl: 0.005 % | 440 | 61 | +++ | 95 | great |

Table 2 indicates that the plant tissue could grow well under the conditions for the test lots 7, 8, 10, 11, 13, 14 and 16, with no contamination or with no adverse drug influence, even when any sterilization process was not carried out.

Table 3 indicates that the plant tissue could grow well and root well at a high yield, under the conditions for the test lots 7, 8, 10, 11, 13, 14 and 16, compared with conventional methods. Additionally, these test lots could be transplanted as they were for acclimatization with no removal of the support materials, so it was found not only that the acclimatization process could be simplified but also that the plant tissue could grow well, compared with conventional methods.

### Plant tissue culture of sweet potato

### Example 4 and Comparative Example 4

Each sweet potato plant with just one node was resected from an aseptically cultured seedling of sweet potato. Five individuals were placed in each plant box. Twenty plant boxes (100 shoots in total) were used per each test lot.

Florialite of Example 1 was used as the support material in the present invention. As a control, Gelrite was used.

For the autoclave lots, the plant tissues were placed aseptically in a clean bench. For the non-autoclave lots, the plant tissues were placed in non-sterile environment (laboratory bench).

Culture was continued for 4 weeks; thereafter, the plants were placed in an acclimatization bed. In case of Gelrite, the plants were placed in a nursery soil after the Gelrite was sufficiently flushed with running water; in case of Florialite, the support material was not rinsed and placed as it was on the acclimatization beds.

Conditions of the plant tissue culture of sweet potato are shown below.
Plant: sweet potato
Support material: Gelrite (2.5 g/l), Florialite (molded product of Example 1; 5 × 5 × 2 cm)
Culture vessel: Magenta Box (one Milliseal membrane attached on the lid part)
Germicide: sodium hypochlorite, iprodion, benomyl
Culture broth: MS culture broth (pH 5.8); 3 % sucrose added to MS culture broth when saccharide is contained
Culturing period: 4 weeks
Exposure to light: 16 hours in light and 8 hours in dark; 100 µmol/m²/sec
CO₂ concentration: 500 µmol/mol
Temperature: 28 °C.

Experimental lots were defined as follows in Table 4.

**Table 4**

| Culture conditions of individual experimental lots |
|---|
| Autoclave Gelrite lots |
| 1. with saccharide |
| 2. non-saccharide |

| Autoclave Florialite lots |
|---|
| 1. with saccharide |
| 2. non-saccharide |

| Non-autoclave Florialite lots |
|---|
| 5. with saccharide |
| 6. non-saccharide |
| 7. non-saccharide/ sodium hypochlorite : 0.005 % (effective chloride concentration) |
| 8. non-saccharide/ sodium hypochlorite : 0.01 % (effective chloride concentration) |
| 9. non-saccharide/ sodium hypochlorite : 0.1 % (effective chloride concentration) |
| 10. non-saccharide/ iprodion : 0.005 % |
| 11. non-saccharide/ iprodion : 0.01 % |
| 12. non-saccharide/ iprodion : 0.2 % |
| 13. non-saccharide/ benomyl : 0.005 % |
| 14. non-saccharide/ benomyl : 0.01 % |
| 15. non-saccharide/ benomyl : 0.2 % |
| 16. non-saccharide/ iprodion : 0.005 %, benomyl :0.005 and sodium hypochlorite : 0.005 %(effective chloride concentration) |

The growth of plant was assayed on the basis of the following items.

### 〈From the start of culture to 4-week later〉

The number of vessels with healthy growth; the number of vessels contaminated with germs; and the number of vessels with adverse drug influence.

### 〈After 4-week culture〉

Fresh weight of plant ; plant length; root growth.

### 〈After transplantation on acclimatization bed〉

Survival ratio and growth.

The measurement was carried out in the same manner as in Example 3, except that the plant length was measured instead of the maximum petiole length. The plant length was measured for each plant, and the average of each experimental lot was calculated. Tables 5 and 6 show the growth results after 4-week culture and acclimatization.

**Table 5**

| Growth state of sweet potato after 4-week culture | | | |
|---|---|---|---|
| Test lots | Healthy vessel | Vessel contaminated with germs | Vessel with adverse drug influence |
| Autoclave Gelrite | | | |
| 1. with saccharide | 20 | 0 | 0 |
| 2. non-saccharide | 20 | 0 | 0 |

| Autoclave Florialite | | | |
|---|---|---|---|
| 3. with saccharide | 20 | 0 | 0 |
| 4. non-saccharide | 20 | 0 | 0 |

| Non-autoclave Florialite | | | |
|---|---|---|---|
| 5. with sacchride | 0 | 20 | 0 |
| 6. non-saccharide | 3 | 17 | 0 |
| 7. non-saccharide NaOCl: 0.005 % | 20 | 0 | 0 |
| 8. non-saccharide NaOCl: 0.01 % | 20 | 0 | 0 |
| 9. non-saccharide NaOCl: 0.1 % | 0 | 0 | 20 |
| 10. non-saccharide iprodion: | 20 | 0 | 0 |
| 0.005 % | 20 | 0 | 0 |
| 11. non-saccharide iprodion: 0.01 % | 0 | 0 | 20 |
| 12. Non-saccharide iprodion: 0.2 % | 20 | 0 | 0 |
| 13. non-saccharide benomyl: 0.005 % | 20 | 0 | 0 |
| 14. non-saccharide benomyl: 0.01 % | 0 | 0 | 20 |
| 15. Non-saccharide iprodion: 0.2 % | 20 | 0 | 0 |
| 16. non-saccharide NaOCl: 0.005 % iprodion: 0.005 %, benomyl: 0.005 % | | | |

**Table 6**

| Growth results after culturing of sweet potato for 4 weeks and subsequent acclimatization | | | | | |
|---|---|---|---|---|---|
| Test lots | Results of culture | | | Results of acclimatization | |
| | Fresh weight of plant in mg | Plant length in mm | Root growth | Survival ratio in % | Growth |
| Autoclave Gelrite | | | | | |
| 1. with saccharide | 300 | 42 | + | 60 | delay |
| 2. non-saccharide | 120 | 24 | ± | 10 | delay |

| Autoclave Florialite | | | | | |
|---|---|---|---|---|---|
| 3. with saccharide | 450 | 58 | ++ | 90 | good |
| 4. non-saccharide | 330 | 50 | ++ | 90 | good |

| Non-autoclave Florialite | | | | | |
|---|---|---|---|---|---|
| 5. with saccharide | - | - | - | - | - |
| 6. non-saccharide | - | - | - | - | - |
| 7. non-saccharide NaOCl: 0.005 % | 480 | 60 | +++ | 95 | great |
| 8. non-saccharide NaOCl: 0.01 % | 460 | 59 | +++ | 95 | great |
| 9. non-saccharide NaOCl: 0.1 % | - | - | - | - | - |
| 10. non-saccharide iprodion: 0.005 % | 520 | 66 | +++ | 95 | great |
| 11. non-saccharide iprodion: 0.01 % | 500 | 64 | +++ | 95 | great |
| 12. Non-saccharide iprodion: 0.2 % | - | - | - | - | - |
| 13. non-saccharide benomyl: 0.005 % | 490 | 61 | +++ | 95 | great |
| 14. non-saccharide benomyl: 0.01 % | 480 | 60 | +++ | 95 | great |
| 15. Non-saccharide benomyl: 0.2 % | - | - | - | - | - |
| 16. non-saccharide NaOCl: 0.005 % iprodion: 0.005%, benomyl: 0.005 % | 470 | 59 | +++ | 95 | great |

Table 5 indicates that the plant tissue could grow well under the conditions for the test lots 7, 8, 10, 11, 13, 14 and 16, with no contamination or no adverse drug influence, even when any sterilization process was not carried out.

Table 6 indicates that the plant tissue could grow well and root well at a high yield, under the conditions for the test lots 7, 8, 10, 11, 13, 14 and 16, compared with conventional methods. Additionally, these test lots could be transplanted as they were for acclimatization with no removal of the support materials, so it was found not only that the acclimatization process could be simplified but also that the plant tissue could grow well, compared with conventional methods.

### Example 5 and Comparative Example 5

As the germicide, KK-681 (K·I Chemical Industry Co.Ltd.), sodium hypochlorite, iprodion and benomyl were used. Experimental lots were defined as follows in Table 7. Other experimental protocols were the same as in Example 4 and Comparative Example 4, then the plant tissues of sweet potato were cultured and assayed. Tables 8 and 9 show the growth results after 4-weeks culture and acclimatization.

**Table 7**

| Culture conditions of individual experimental lots |
|---|
| Autoclave Gelrite lots |
| 1. with saccharide. |
| 2. non-saccharide |

| Autoclave Florialite lots |
|---|
| 1. with saccharide |
| 2. non-saccharide |

| Non-autoclave Florialite lots |
|---|
| 5. with saccharide |
| 6. non-saccharide |
| 7. non-saccharide/ KK-681 : 0.01 % |
| 8. non-saccharide/ KK-681 : 0.5 % |
| 9. non-saccharide/ KK-681 : 2.0 % |
| 10. non-saccharide/ KK-681 : 0.005 %, sodium hypochlorite : 0.005 % (effective chloride concentration) iprodion : 0.005 % and benomyl : 0.005 % |

**Table 8**

| Growth state of sweet potato after 4-week culture | | | |
|---|---|---|---|
| Test lots | Healthy vessel | Vessel contaminated with germs | Vessel with adverse drug influence |
| Autoclave Gelrite | | | |
| 1. with saccharide | 20 | 0 | 0 |
| 2. non-saccharide | 20 | 0 | 0 |

| Autoclave Florialite | | | |
|---|---|---|---|
| 3. with saccharide | 20 | 0 | 0 |
| 4. non-saccharide | 20 | 0 | 0 |

| Non-autoclave Florialite | | | |
|---|---|---|---|
| 5. with saccharide | 0 | 20 | 0 |
| 6. non-saccharide | 3 | 17 | 0 |
| 7. non-saccharide KK-681 :0.01 % | 20 | 0 | 0 |
| 8. non-saccharide KK-681 : 0.5 % | 20 | 0 | 0 |
| 9. non-saccharide KK-681 : 2.0 % | 0 | 0 | 20 |
| 10. non-saccharide KK-681 : 0.005 % NaOCl : 0.005 %, iprodion : 0.005 %, benomyl : 0.005 % | 20 | 0 | 0 |

**Table 9**

| Growth results after culturing of sweet potato for 4 weeks and subsequent acclimatization | | | | | |
|---|---|---|---|---|---|
| Test lots | Results of culture | | | Results of acclimatization | |
| | Fresh weight of plant in mg | Whole plant length in mm | Root growth | Survival ratio in % | Growth |
| Autoclave Gelrite | | | | | |
| 1. with saccharide | 300 | 42 | + | 60 | delay |
| 2. non-saccharide | 120 | 24 | ± | 10 | delay |

| Autoclave Florialite | | | | | |
|---|---|---|---|---|---|
| 3. with saccharide | 450 | 58 | ++ | 90 | good |
| 4. non-saccharide | 330 | 50 | ++ | 90 | good |

| Non-autoclave Florialite | | | | | |
|---|---|---|---|---|---|
| 5. with saccharide | - | - | - | - | - |
| 6. non-saccharide | - | - | - | - | - |
| 7. non-saccharide KK-681 :0.01 % | 500 | 63 | +++ | 95 | great |
| 8. non-saccharide KK-681 : 0.5 % | 490 | 62 | +++ | 95 | great |
| 9. non-saccharide KK-681 : 2.0 % | - | - | - | - | - |
| 10. non-saccharide KK-681 : 0.01 %, NaOCl: 0.005 %, iprodion: 0.005%, benomyl: 0.005 % | 465 | 59 | +++ | 95 | great |

Table 8 indicates that the plant tissue could grow well under the conditions for the test lots 7, 8 and 10, with no contamination or no adverse drug influence, even when any sterilization process was not carried out.

Table 9 indicates that the plant tissue could grow well and root well at a high yield, under the conditions for the test lots 7, 8 and 10, compared with conventional methods. Additionally, these test lots could be transplanted as they were for acclimatization with no removal of the support materials, so it was found not only that the acclimatization process could be simplified but also that the plant tissue could grow well, compared with conventional methods.

### Environmental conditions of gas and light

### Example 6 and Comparative Example 6

In Example 6, the photosynthetic photon flux was 200 µmol/m²/sec and the carbon dioxide gas concentration was 500 µmol/mol; in Comparative Example 6, the photosynthetic photon flux was 30 µmol/m²/sec and the carbon dioxide gas concentration was 250 µmol/mol. Other experimental protocols were the same as in Example 4 and Comparative Example 4.

Under the following conditions, sweet potato plant tissue was cultured.
Plant: sweet potato
Support material: Florialite (molded product of Example 1; 5 × 5 × 2 cm)
Culture vessel: Magenta Box (one Milliseal membrane attached on the lid part)
Germicide: iprodion 0.01 %
Culture broth: MS culture broth (pH 5.8, non-saccharide)
Culturing period: 4 weeks
Exposure to light: 16 hours in light and 8 hours in dark; 30 µmol/m²/sec, 200 µmol/m²/sec
CO₂ concentration: 250 µmol/mol, 500 µmol/mol
Temperature: 28 °C

Experimental lots were defined as follows in Table 10.

**Table 10**

| Culture conditions of individual experimental lots Non-autoclave Florialite lots |
|---|
| 1. photosynthetic photon flux at 30 µmol/m²/sec and CO₂ concentration at 250 µmol/mol. |
| 2. photosynthetic photon flux at 200 µmol/m²/sec and CO₂ concentration at 500 µmol/mol. |

The growth of plant was assayed on the basis of the following items.

### 〈After 4-week culture〉

Fresh weight of plant; plant length; root growth.

### 〈After transplantation on acclimatization bed〉

Survival ratio and growth.

The measurement was carried out in the same manner as in Example 4. Table 11 shows the growth results after 4-week culture.

**Table 11**

| Test lots | Results of culture | | | Results of acclimatizati on | |
|---|---|---|---|---|---|
| | Fresh weight of plant in g | Plant length in mm | Root growth | Survival ratio in % | Growth |
| Non-autoclave Florialite | | | | | |
| 1. photosynthetic photon flux at 30 µmol/m²/sec and CO₂ concentration at 250 µmol/mol | 0.4 | 55 | ++ | 95 | good |
| 2. photosynthetic photon flux at 200 µmol/m²/sec and CO₂ concentration at 500 µmol/mol | 0.7 | 70 | +++ | 100 | great |

Table 11 shows that the gas and light conditions for the experimental lot 2 serve for excellent growth of the plant tissue.

As apparently shown in the Examples, the method for plant tissue culture of the present invention can cut out laborious sterilization procedures of the culture medium or culture vessel or the like and doesn't require the sterile transplantation of the plant tissue. Fourthermore root growth and growth of the plant are excellent at a high yield. The resulting plant can be transplanted as it is with no removal of the support material for acclimatization, whereby the acclimatization process can be simplified or cutted out to shorten the period for growing into mature seedling, so that a vast amount of seedlings can be obtained at one time without employing culture vessels. Thus, the method for plant tissue culture of the present invention is extremely advantageous, compared with the conventional methods.

This invention being thus described, it will be obvious that the same may be varied in various ways. Such variations are not to be regarded as departure from the spirit and scope of the invention, and all such modifications would be obvious for one skilled in the art intended to be included within the scope of the following claims.

## Claims

1. A method for plant tissue culture comprising the step of culturing plant tissue by using a plant tissue culture support material with density of 0.01 to 2 g/cm³ which is comprised of vermiculite of average particle diameter of 0.1 to 10 mm and cellulose fiber of average fiber length of 0.01 to 5 mm at a mixing ratio in weight of the vermiculite and the cellulose fiber being 5 : 95 to 95 : 5, and a culture broth with addition of a germicide at such an amount that the germicide itself can singly prevent contamination.

2. A method for plant tissue culture according to claim 1, wherein the culture broth does not contain carbon source.

3. A method for plant tissue culture according to claim 1 or 2, wherein the germicide is a mixture of one or two or more selected from the group consisting of chlorine germicides and azotic germicides.

4. A method for plant tissue culture according to any one of claims 1 to 3, wherein the germicide is a mixture of one or two or more selected from the group consisting of hypochlorite salts, iprodion and benomyl.

5. A method for plant tissue culture according to claim 4, wherein effective concentration of the hypochloric acid is 0.001 to 0.05 %, concentration of the iprodion is 0.001 to 0.1 % and that of the benomyl is 0.001 to 0.1 %.

6. A method for plant tissue culture according to any one of claims 1 to 5, the step of culturing plant tissue is performed under light at a photosynthetic photon flux of 50 to 500 µmol/m²/sec while maintaining carbon dioxide gas concentration in culture environment to be at 300 to 2000 µmol/mol.
